# EUROPEAN PATENT SPECIFICATION

(11) **EP 2 706 907 B1**
(45) Date of publication and mention of the grant of the patent: **06.10.2021**
(21) Application number: 12782902.6
(22) Date of filing: 14.05.2012
(51) Int. Cl.: A61B 5/021, A61B 5/02, A61B 5/0205, A61B 5/03, A61B 5/00, A61B 5/08

(54) **AUTOMATED PROCESS FOR USE IN ASSESSING CARDIAC FILLING PRESSURE NON-INVASIVELY**
AUTOMATISCHES VERFAHREN ZUR VERWENDUNG IN DER NICHTINVASIVEN MESSUNG DES KARDIALEN FÜLLUNGSDRUCKS
PROCESSUS AUTOMATISÉ À UTILISER DANS L'ÉVALUATION DE LA PRESSION DE REMPLISSAGE CARDIAQUE DE MANIÈRE NON INVASIVE

(30) Priority: 12.05.2011 US 201161485334 P
(43) Date of publication of application: 19.03.2014
(73) Proprietor: The Johns Hopkins University, Baltimore, MD 21218 (US)
(72) Inventor: SILBER, Harry A., Owings Mills Maryland 21117 (US)
(74) Representative: Murgitroyd & Company
(86) International application number: PCT/US2012/037771
(87) International publication number: WO 2012/155133

(56) References cited:
- WO-A1-2011/051819
- WO-A2-2010/005536
- US-A1- 2003 097 158
- US-B2- 6 832 113
- US-B2- 7 195 594
- C. GILLARD ET AL: "Operating characteristics of the finapress system to predict elevated left ventricular filling pressure", CLINICAL CARDIOLOGY, vol. 29, no. 3, 1 March 2006 (2006-03-01), pages 107-111, XP055141029, ISSN: 0160-9289, DOI: 10.1002/clc.4960290305
- D. WEILENMANN ET AL: "Noninvasive Evaluation of Pulmonary Capillary Wedge Pressure by BP Response to the Valsalva Maneuver*", CHEST, vol. 122, no. 1, 1 July 2002 (2002-07-01), pages 140-145, XP055141030, ISSN: 0012-3692, DOI: 10.1378/chest.122.1.140
- CANNESSON MAXIME ET AL: "Relation between respiratory variations in pulse oximetry plethysmographic waveform amplitude and arterial pulse pressure in ventilated patients", CRITICAL CARE, BIOMED CENTRAL LTD., LONDON, GB, vol. 9, no. 5, 23 August 2005 (2005-08-23) , pages R562-R568, XP021012397, ISSN: 1364-8535, DOI: 10.1186/CC3799
- BOSSEAU MURRAY W ET AL: "THE PERIPHERAL PULSE WAVE: INFORMATION OVERLOOKED", JOURNAL OF CLINICAL MONITORING, BOSTON, MA, US, vol. 12, 1 January 1996 (1996-01-01), pages 365-377, XP001040838, DOI: 10.1007/BF02077634
- None

## Description

### FIELD OF THE INVENTION

The present invention relates generally to cardiac care. More particularly, the present invention relates to a method for assessing cardiac filling pressure.

### BACKGROUND OF THE INVENTION

Heart failure is a prevalent diagnosis and is increasing at an annual growth rate of 10.5%. Additionally, more than 1 million hospital discharges per year have a primary diagnosis of heart failure, the most common discharge diagnosis. Patients admitted to the hospital with Congestive Heart Failure (CHF) symptoms from severe fluid overload usually have had fluid build-up without symptoms for days or weeks prior to admission. Detection of elevated fluid prior to symptoms could prevent admissions for "acute" decompensated heart failure. Also, many patients with CHF are inadequately diuresed at discharge (i.e., not enough fluid has been removed prior to discharge). This typically leads to re-admission of the patient for CHF. The rate of rehospitalization for heart failure is very high; about 20% at 30 days after discharge, and about 30% at 60-90 days after discharge.

As is known to those skilled in the art, measurement of left ventricular filling pressure (LVFP) allows detection of CHF and helps assessment of the efficacy of therapy for CHF. Left ventricular pressure can be measured directly by placing a catheter in the left ventricle to obtain the end diastolic pressure (LVDEP) or indirectly by placing a catheter in the pulmonary artery to measure the pulmonary capillary wedge pressure (PCWP). It should be noted that measuring LV filling pressure using a catheter is generally considered the gold standard. The pulmonary artery catheterization (PAC) technique that is used to measure either LVFP or PCWP is considered an invasive surgical procedure. Thus, there are attendants' risks and costs associated with such a technique and the reported complications include catheter migration, arrhythmias, pulmonary artery rupture, thrombosis, infection, bleeding and pneumothorax.

Physical examination of a patient has poor sensitivity for detecting elevated cardiac filling pressure, especially in patients with chronic heart failure. Some common aids use to help in the diagnosis of elevated LVFP include chest X-ray, serum biomarkers (BNP, pro BNP) and echocardiography. These techniques have important practical limitations. Also, clinical and radiographic methods of detecting CHF are generally insensitive to alterations in LVFP.

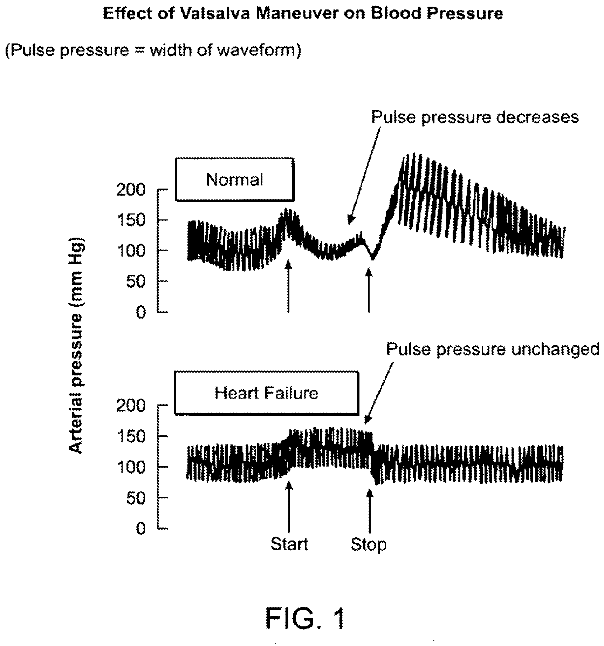

There have been attempts made to develop a non-invasive method and system for estimating LVFP to allow for early detection and treatment of CHF, which has been shown to reduce the rate of hospitalization and mortality. A non-invasive method of assessing PCWP has been reported that uses the strain phase of the Valsalva maneuver. In a Valsalva maneuver, the subjects or patients perform a forced expiration into a closed tube or strain against a closed glottis. This results in a unique, well-described cardiovascular response, involving both a rise and fall in arterial pressure and a momentary opposite-direction change in right and left heart stroke volume. The effect of the Valsalva maneuver on blood pressure for a normal heart and a heart diagnosed with heart failure is shown in FIG. 1.

In this method a non-invasive pulmonary tonometer and a digital pulmonary monitor is used to continuously acquire arterial and expiratory pressure signals during a Valsalva maneuver. A software program also is used to analyze the arterial pressure signals and derive a LVEDP using a predictive algorithm. The tonometer is not easy to use and is located on the wrist or finger. While this system is not yet commercially available it is expected to cost in the tens of thousands of dollars. The tonometer also is not as widely used a technology as compared to some other medical devices.

Another reported non-invasive technique is referred to as impedance cardiography (ICG). In the ICG technique, four electrodes are placed around the neck and lower thorax and four electrodes are placed on the lateral surface of the abdomen. In this arrangement the outer sensors transmit current and the inner sensors measure impedance. The impedance being measured across the chest is related to fluid volume; thus it can be used to measure changes to fluid volume. However, such measurements do not reflect absolute measurements of LVEDP. The cost for such a device also is in the tens of thousands.

Reported in US2003/097158 is a non-invasive method and apparatus for monitoring the condition of a heart failure patient and for optimizing the pacing parameters of a cardiac device implanted in a patient.

Gillard et al; Clin Cardiol. 2006 Mar; 29(3): 107-11 (operating characteristics of the Finapress system to predict elevated left ventricular filling pressure) discloses that the PAR determined by the Finapress procedure may be a useful bedside diagnostic tool in patients with cardiac conditions.

Weilenmann et al; Chest. 2002 Jul; 122(1): 140-5 (Noninvasive evaluation of pulmonary capillary wedge pressure by BP response to the Valsalva maneuver) discloses that PCWP and changes during therapy can be estimated noninvasively by measuring the PAR during the VM with acceptable accuracy in stable patients with cardiac conditions.

Maxime Cannesson et al; Critical Care 2005, 9:R562-R568 (Relation between respiratory variations in pulse oximetry plethysmographic waveform amplitude and arterial pulse pressure in ventilated patients) discloses that respiratory variation in arterial pulse pressure above 13% can be accurately predicted by a respiratory variation in POP waveform amplitude above 15%. This index has potential applications in patients who are not instrumented with an intra-arterial catheter.

Bosseau Murray et al; Journal of Clinical Monitoring 10/1996; 12(5):365-77 (The peripheral pulse wave: information overlooked) discloses that although the waveform derived from a peripheral pulse monitor or pulse oximeter may resemble an arterial pressure waveform, it is in fact a visualization of blood volume change in transilluminated tissue caused by passage of blood: an indication of perfusion or blood flow. The paper speculates that new physiological tests of autonomic function and cardiac preload could be developed using pulse plethysmography Document WO2010/005536 A2 discloses determining cardiac filling pressure non-invasively during a Valsalva maneuver based on a photoplethysmography signal.

Thus, there is a continuing need to develop non-invasive systems and methods for assessing cardiac filling pressure, more specifically LV filling pressure. It also would be desirable to provide such a device that provides similar useful clinical information as prior art systems and methods. Such systems preferably would be less costly than prior art systems, would use a robust transducer and such methods would not involve or require a greater skill set than that required for user of prior art methods. Moreover, it would be desirable to provide a device or system whose operation is automated and therefore simple enough for a patient or the like to use at home.

### SUMMARY OF THE INVENTION

The foregoing needs are met, to a great extent, by the present invention, which provides for a method and apparatus according to the independent claims. Other aspects and embodiments are defined by the dependent claims and discussed in detail below.

In one aspect a method for automating a process for determining cardiac filling pressure non-invasively includes analysing a photoplethysmography signal having a pulse amplitude in accordance with the independent claim 1. The analysis is done to determine a point in time that the pulse amplitude of the photoplethysmography signal does not vary by a predetermined amount over a first predetermined time period. The method also includes displaying an instruction on a user interface to inform a user that an instruction to initiate an expiratory effort will be displayed. An instruction is displayed on the user interface informing the user to begin an expiratory effort. The expiratory effort of the user is compared to a predetermined goal range for expiratory effort, and an indicator of the user's expiratory effort relative to the predetermined goal range is displayed on the user interface.

In accordance with the disclosure of the present invention a method for automating a process for determining cardiac filling pressure non-invasively includes programming a computer readable medium to execute steps for the method. These steps include analyzing a photoplethysmography signal having a pulse amplitude. The analysis is done to determine a point in time that the pulse amplitude of the photoplethysmography signal does not vary by a predetermined amount over a first predetermined time period. Another step includes transmitting a first instruction to be displayed on a user interface, said instruction informing a user that an instruction to initiate an expiratory effort will be displayed. The method also includes transmitting a second instruction to be displayed on the user interface, said instruction informing the user to begin an expiratory effort. The expiratory effort of the user is compared to a predetermined goal range for expiratory effort. Additionally, a third instruction is transmitted to be displayed on the user interface, said instruction indicating the user's expiratory effort relative to the predetermined goal range on the user interface.

In accordance with the disclosure, the method further includes instructing the user to sustain the expiratory effort for a second predetermined period of time. The method can also include displaying an elapsed time for the expiratory effort as well as a time remaining in the second predetermined period of time.

It is indicated to the user that the data acquisition sequence is invalid, when the expiratory effort does not meet the predetermined goal range within a third predetermined period of time. It is also indicated to the user that the data acquisition sequence is invalid when the expiratory effort exceeds the predetermined range for expiratory effort. After the predetermined amount of time has elapsed it is displayed that the user can cease the expiratory effort. A first average of the pulse amplitude of the photoplethysmography signal is calculated for a fourth predetermined period of time before the user ceases the expiratory effort. A second average of the pulse amplitude of the photoplethysmograpy signal is also calculated for a fifth period of time before an initiation of the expiratory effort. Additionally, a pulse amplitude ratio of the first average of the pulse amplitude to the second average of the pulse amplitude is calculated. These steps can also be programmed into the computer or computer-readable medium.

In accordance with another aspect of the disclosure, an apparatus for automating a process for determining cardiac filling pressure non-invasively, includes a device including a housing, said device having a user interface disposed within said housing. The device also includes an expiratory effort reception apparatus being coupled to said housing. A photoplethysmograph is also coupled to the housing. A computer readable medium is also included and is programmed to execute steps to determine cardiac filling pressure. The computer readable medium is programmed to analyze a signal from the photoplethysmograph. The signal has a pulse amplitude, and the analysis is done to determine a point in time that the pulse amplitude of the signal does not vary by a predetermined amount over a first predetermined time period. The computer readable medium is also programmed to transmit a first instruction to be displayed on a user interface, said instruction informing a user that an instruction to initiate an expiratory effort will be displayed. A second instruction is transmitted to be displayed on the user interface, said instruction informing the user to begin an expiratory effort. The expiratory effort of the user to a predetermined goal range for expiratory effort is calculated. Additionally, a third instruction is transmitted to be displayed on the user interface, said instruction indicates the user's expiratory effort relative to the predetermined goal range on the user interface.

### BRIEF DESCRIPTION OF THE DRAWINGS

The accompanying drawings provide visual representations which will be used to more fully describe the representative embodiments disclosed herein and can be used by those skilled in the art to better understand them and their inherent advantages. In these drawings, like reference numerals identify corresponding elements and:
FIG. 1 illustrates a graphical view illustrating the effect of the Valsalva maneuver on blood pressure for a normal heart and a heart with heart failure.
FIG. 2 illustrates a high level flow diagram of a methodology for assessing cardiac filling pressure non-invasively.
FIG. 3A illustrates a block diagram of an exemplary system for assessing cardiac filling pressure non-invasively according to the present invention.
FIG. 3B illustrates a block diagram of another exemplary system for assessing cardiac filling pressure non-invasively according to the present invention.
FIG. 3C illustrates a block diagram of yet another exemplary system for assessing cardiac filling pressure non-invasively.
FIG. 3D illustrates a block diagram of yet another exemplary system for assessing cardiac filling pressure non-invasively.
FIGS. 3E-H illustrate various block diagrams illustrating a device used in various communication environments.
FIG. 4 illustrates graphical view of a photoplethysmography signal and of invasively measured blood pressure during the Valsava maneuver.
FIG. 5 illustrates a graphical view of a photoplethysmography waveform and the expiratory pressure waveform for a heart with a normal filling pressure using the system of the present invention.
FIG. 6 illustrates a graphical view of a photoplethysmography waveform and the expiratory pressure waveform for a heart with an elevated filling pressure using the system of the present invention.
FIG. 7 illustrates a graphical view of a photoplethysmography waveform and the expiratory pressure waveform for a heart of a patient that had been initially diagnosed as being fluid overloaded, but which it was later determined that the heart had a normal filling pressure using the system of the present invention.
FIG. 8 illustrates a graphical view of LVEDP by catheter versus Average Pulse Amplitude Ratio for a patient study, where the pulse amplitude ratios were determined using the system of the present invention.
FIG. 9 illustrates a flow chart of a method of automation for determining cardiac filling pressure non-invasively.
FIG. 10 illustrates a diagram of a method of automation for determining cardiac filling pressure non-invasively.

### DETAILED DESCRIPTION

The presently disclosed subject matter now will be described more fully hereinafter with reference to the accompanying Drawings, in which some, but not all examples of the present disclosure are shown. Like numbers refer to like elements throughout. The presently disclosed subject matter may be embodied in many different forms and should not be construed as limited to the embodiments set forth herein; rather, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions and the associated Drawings. Therefore, it is to be understood that the presently disclosed subject matter is not to be limited to the specific embodiments disclosed and that modifications and other embodiments are intended to be included within the scope of the appended claims.

An embodiment in accordance with the present disclosure provides an automated device and method for determining cardiac filling pressure non-invasively. The device includes a computer readable medium programmed to analyze a photoplethysmography signal having a pulse amplitude. The analysis is done to determine a point in time that the pulse amplitude of the photoplethysmography signal does not vary by a predetermined amount over a first predetermined time period. The method also includes displaying instructions for the user to prepare for and begin an expiratory effort. The expiratory effort of the user is compared to a predetermined goal range for expiratory effort, and an indicator of the user's expiratory effort relative to the predetermined goal range is displayed on the user interface.

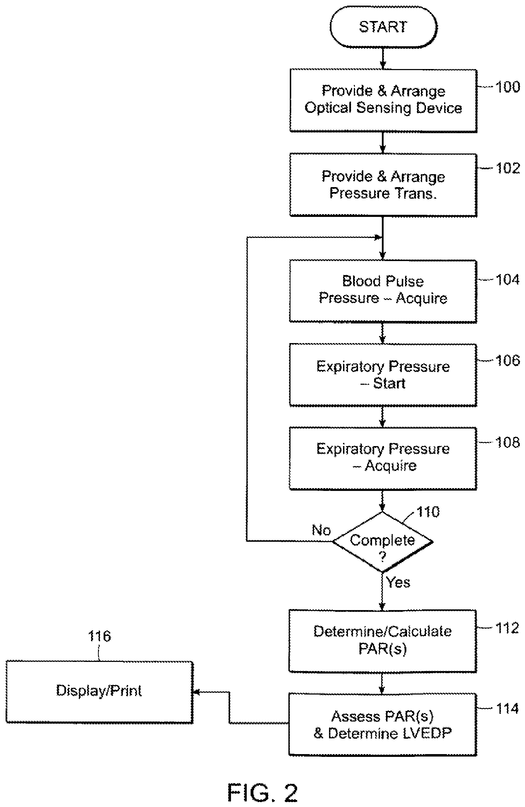

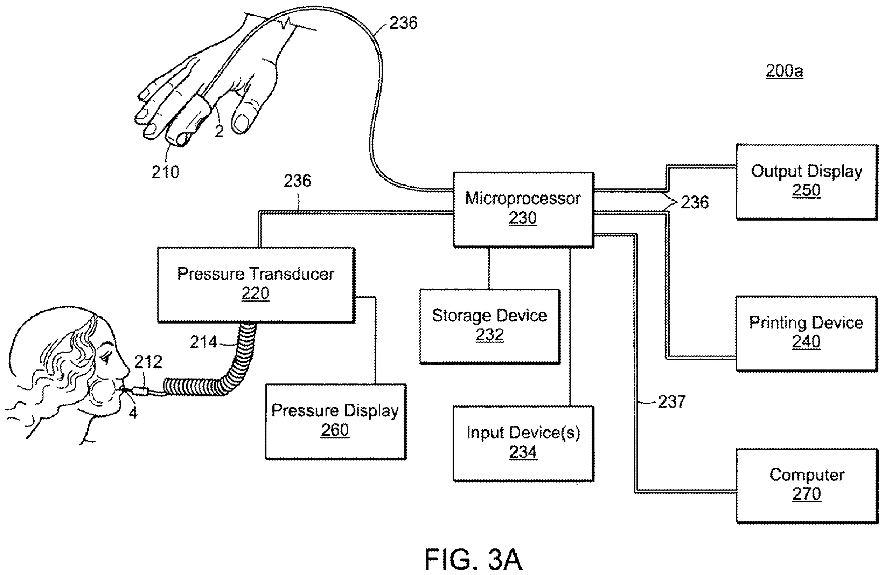

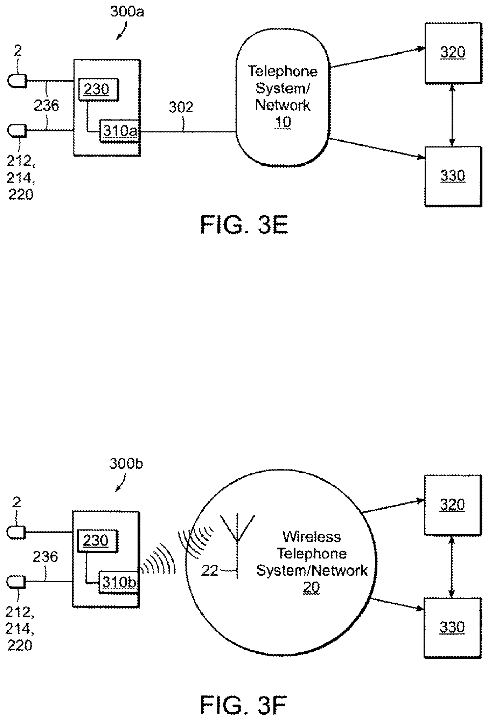

FIG. 2 illustrates a high level flow diagram of a methodology for assessing cardiac filling pressure non-invasively according to the present invention. Reference also should be made to FIGS. 3A-D which illustrate various block diagrams of systems 200a-d for assessing cardiac filling pressure non-invasively that can include details and features not shown in FIG. 2. Reference also should be made to FIGS. 3E-H which are various block diagrams illustrating various ways in which a system 200 or a device embodying such a system (whole or in part) along with one of a number of communication techniques whereby the system or device can transmit or communicate information to for example, the practitioner or a monitoring service. Before describing the methodology, the illustrative composition of such each system 200a-d of the present invention is first described.

FIG. 3A illustrates a block diagram of a device 200a according to an embodiment of the invention. The device 200a includes an optical pulse volume sensing device 210 and a pressure transducer 220. A means for determining a pulse amplitude ratio uses the pulse amplitude as determined from output signals from the optical pulse volume sensing device. The measure of the pulse amplitude is taken near the end of the expiratory effort as determined from the outputs from the pressure transducer and a baseline pulse amplitude as determined from output signals from the optical pulse volume sensing device. The device also includes a means for assessing the determined pulse amplitude ratio so as to determine a filling pressure condition for the heart of the patient.

Also as illustrated in FIG. 3A, the optical pulse volume sensing device 210 is configured and arranged so it can be located on, and removably secured to, a finger 2 or digit of a patient or subject and so that the optical pulse volume sensing device senses a change in volume cause by the pressure pulse. The pressure transducer 220 is configured and arranged to be fluidly coupled to a mouth 4 of the patient, such that the pressure transducer measures expiratory pressure of the patient. Preferably, the optical pulse volume sensing device 210 is a photoplethysmography (PPG) transducer and the pressure transducer 220 is any one of a number of devices known to those skilled in the art and appropriate for the intended use.

The pressure transducer 210, illustrated in FIG. 3A, is operably coupled to a mouth piece 212 and/or tubing 214, so that the pressure transducer is remote from the patient's mouth 4. One end of the mouth piece 212 or the tubing 214 is fluidly coupled to the mouth. Preferably, one end of the mouth piece 212 is removably, fluidly coupled to the mouth using any of a number of techniques known to those skilled in the art, and the other end of the mouth piece and an end of the tubing are fluidly coupled or joined to each other to form a unitary structure.

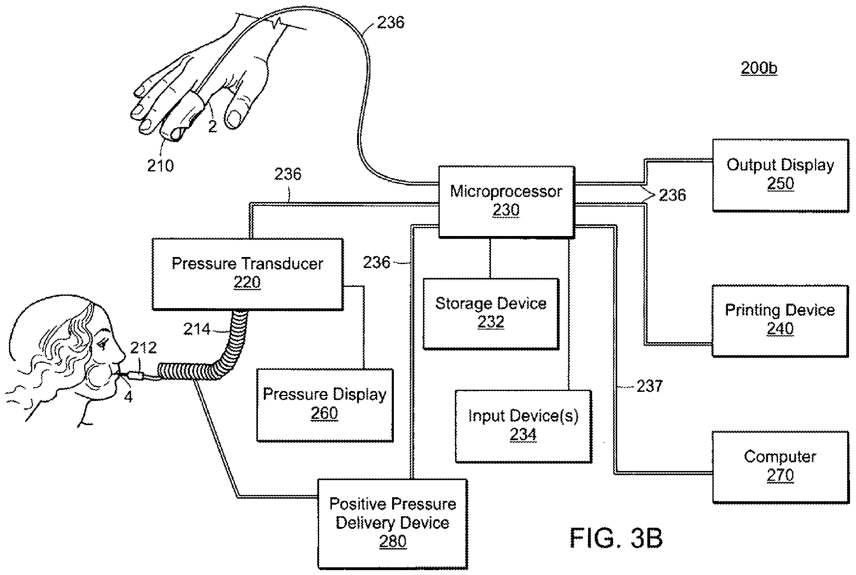

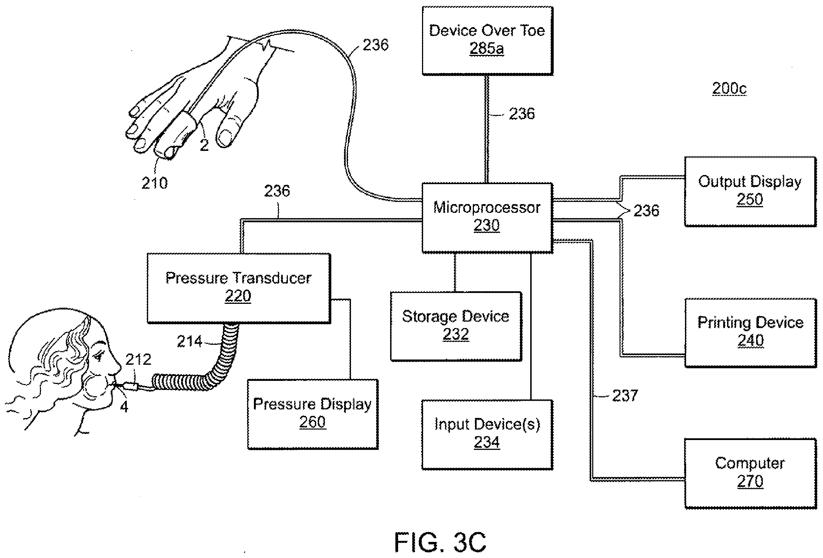

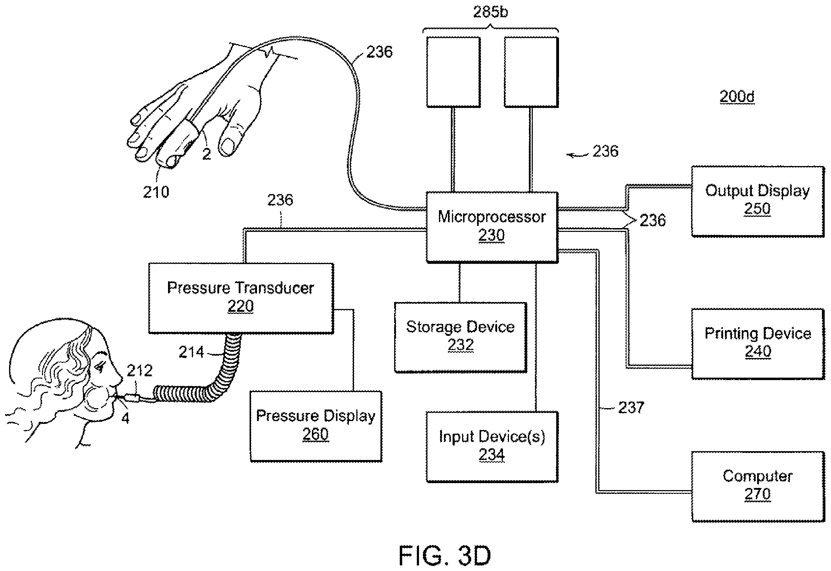

As illustrated in FIG. 3B, there is shown another system 200b according to the present invention that is configured and arranged so as to include a positive pressure delivery device 280 that is selectively fluidly coupled to the tubing 214 and operably and/or communicatively coupled to the microprocessor 230. The microprocessor 230 controls the positive pressure delivery device 280, so that a pressurized breathing gas mixture (e.g., air) is delivered to the patient via the tubing 214 for a predetermined period of time. Suggested times for the expiratory effort are described herein. The microprocessor 230 controls the positive pressure delivery device 280, so that a pressurized breathing gas mixture of different pressures is delivered to the patient. The positive pressure delivery device 280 includes a source of the breathing mixture (e.g., tank, or connection to a delivery system) and a valve that is interposed between the breathing mixture source and the tubing 214. The valve is operably coupled to the microprocessor 230, whereby the microprocessor controls the valve for selectively delivering the breathing mixture to the patient. In further embodiments, the valve also is of the type that is controllable so as to deliver gas mixtures at different pressures. FIGS. 3C, D illustrate exemplary systems 200b-c that further include a device 285a,b that allows the acquisition of information that the clinician can use to assess or determine the arterial stiffness or central arterial stiffness (e.g., the pulse wave velocity, PWV) of the patient. Some studies have shown that central arterial stiffness can affect the Pulse Amplitude Ratio. Thus, taking into account central arterial stiffness can improve the accuracy of the device for evaluating cardiac filling pressure. The device can take the form of another photoplethysmography (PPG) transducer 285a, as illustrated in FIG. 3C, that is located on or attached to a toe of the patient, but any suitable device for the purpose could be used. An ECG signal lead 285b, as illustrated in FIG. 3D, is attached to one finger of each hand. Either of these two provided outputs can be used in combination with any of a number of techniques known to those skilled in the art to provide a mechanism by which the clinician can ascertain the central arterial stiffness of the patient.

The means for determining a pulse amplitude ratio and the means for assessing the determined pulse amplitude ratio are embodied in a microprocessor **230** that is communicatively coupled to each of the pressure transducer **220** and the optical pulse volume sensing device **210**. The functionalities are coupled by cables **236**, as is known to those skilled in the art and appropriate for the intended use (e.g., electrical cables, optical cables). Such a microprocessor **230** includes random access memory, cache and other functionalities (not shown) that allow the microprocessor to function in the intended manner. The microprocessor **230** is embodied in any of a number of computers, as are known to those skilled in the art.

An applications program for execution on the microprocessor **230** is provided, as well. The applications program controls operation of the system and the microprocessor. It is within the skill of those knowledgeable in the computer or software arts to develop such an applications program based on the disclosure herein, including the following discussion regarding the methodology of the present invention. In particular, the applications program includes program or code segments and instructions and criteria for determining the pulse amplitude ratio from the output signals from the pressure transducer and the optical pulse volume sensing device.

One or more storage devices **232** are operably coupled to the microprocessor **230** for storage of, for example, the applications program, the operating system and any data or input information as directed by the user and/or the applications program. Such a storage device is any of a number of devices known to those skilled in the art and includes magnetic hard drives, and flash storage devices that embody flash or non-volatile flash memory. Also operably coupled to the microprocessor **230** is/are one or more input devices **234** such as a keyboard and/or mouse that allow the user to control operation of the microprocessor.

In the case where the microprocessor **230** is not embodied in a computer, the microprocessor **230** is further configured and arranged so it can be operably coupled to a computer **270**, for example via removable cables **237** (e.g., USB cables). In this way, information temporarily stored in the storage device **232** can be uploaded to the computer for further analysis and/or long term storage or to allow communication of data and information to another via a network, an external communication system or via a recording medium (e.g., optical disk, magnetic recording medium).

In further embodiments, the microprocessor **230** also is operably coupled to an output display **250** and a printing device **240**. The output display **250** can take the form of any of a number of display devices as are known to those skilled in the art or hereinafter developed that are appropriate for inter connection with a microprocessor and for displaying information thereon that is communicated from the microprocessor. The printing device **240** can take the form of any of a number of devices known to those skilled in the art or hereinafter developed that are appropriate for inter connection with a microprocessor and for printing information there from and communicated from the microprocessor.

In embodiments or aspects of the present disclosure, the microprocessor **230** and other functionalities of the systems **200***a-d* are intended to be disposed within any of a number of enclosures known to those skilled in the art to form a device that can be configured to have varying degrees of portability, for example, from being located on a rolling cart to being put in the coat of the medical personnel using the device. As such, the output display **250** and/or printing device **240** also can be any of a number of devices that can be co-located in such an enclosure with the microprocessor **230** and other functionalities of the system **200***a-d.* Preferably, to increase portability for home and patient use, the enclosure and display are integrated and are portable in size. There are a number of devices or existing platforms known to those skilled (e.g., Wellch Allyyn Spot Vital Signs Model 42NOB-E1) that can be configured to acquire patient data and are operably couplable to an optical pulse volume sensing device. Thus, it also is contemplated to adapt such existing platforms or devices so as to carry out any functions described herein for the systems **200***a-d* of the present invention.

The system of the present disclosure may also be configurable so that it is free-standing, on a rolling cart, or portable. Such a system is also configurable so that it can be used in an outpatient clinic, an emergency department, a medical ward, an intensive care unit, skilled care facilities, assisted living facilities, or home or used by home services such as for example a visiting nursing service.

The system **200***a-d* further includes a pressure display **260** that is operably coupled to the pressure transducer **220** to provide a local display of the expiratory pressure/pressurized inspiratory pressure. Such a display is used by the medical personnel and/or the patient to determine that the expiratory pressure of the patient is at the value desired for a given measurement process. Thus, the pressure display **260** is any of a number of digital or analog displays or pressure measurement devices that are appropriate for the intended use and anticipated pressures or pressure ranges. In more particular embodiments, the pressure display **260** is configurable to further provide a display of elapsed time.

The device can include an enclosure configured to from a portable device, where the device is further configured and arranged so as to be in a form that is usable by a patient or other persons that do not have specific medical training outside a clinical setting (e.g., the home of a patient) and without the aid or guidance of a clinical practitioner or other medical personnel, or the device is usable by medical personnel (e.g., visiting nurse or nursing aid) outside the clinical setting (e.g., patient's home). For example, the device is preferably configured to execute the method described below, such that the device provides a simple automated method of assessing cardiac filling pressure non-invasively.

The device is configured and arranged with one or more communication interfaces **310**, illustrated in FIGS. 3E-3H, so that the user of the device (e.g., patient, practitioner, visiting nurse, technician, clinical personnel) can easily transmit or communicate the data acquired and/or determined using the device (i.e., acquired information), directly or ultimately to the practitioner who should receive such clinical information. For example, the information being transmitted can be communicated directly to a computer or storage device under the control of the practitioner or to a monitoring service that subsequently transmits the acquired information to the practitioner also using any of a number of techniques known to those skilled in the art. In another example, data can be acquired by a monitoring service in a clinical setting or from patient's home and then communicated to the practitioner or their office.

FIGS. 3E-H also illustrate that the one or more communication interfaces **310***a-d* provide a mechanism by which the device **300***a-d* can be communicatively coupled to any of a number of communication systems known or hereinafter created so that the acquired information can be transmitted or communicated over such a communication system.

More particularly, FIG. 3E illustrates one exemplary device **300***a* according to the present invention that includes an interface **310***a*, such as a modem or equivalent, which is operably coupled to the microprocessor **230** and to a telephone phone system **10** via a hard land line **302** (e.g., copper, optical). As is known to those skilled in the art, such an interface **310** is configured and arranged so that the microprocessor can communicate information over the telephone system **10** to another device that is similarly operably coupled to the telephone system. In this way, the clinical information obtained using the device **300***a* can be communicated to one or both of the practitioner **320** or a monitoring service **330** via the telephone system. As illustrated in FIG. 3E, the monitoring service is communicatively coupled to the practitioner using any of a number of communication techniques know to those skilled in the art.

FIG. 3F illustrates a second exemplary device **300***b* that includes an interface **310***b*, as is known to those skilled in the telephone arts for wirelessly (e.g., using RF transmissions) coupling the second exemplary device **300***b* to a wireless telephone system **20**. As is known to those skilled in the art, such a wireless telephone system **20** includes an antenna **22** or tower that receives the signals from a wireless device and also can be coupled to a wired telephone system or another wireless telephone system so as to allow a communication link between the second exemplary device **300***b* and another device. Such an interface **310***b* is operably coupled to the microprocessor **230** and generally includes an antenna so that wireless (RF) signals pass between the wireless telephone phone system **20** and the exemplary second device **300***b*.

Such a wireless interface **310***b* allows the microprocessor **230** to communicate information via RF transmission to/from the telephone system **20** and thus onto another device that is similarly operably coupled to the telephone system. In this way, the clinical information obtained using the device **300***b* can be communicated to one or both of the practitioner **320** or a monitoring service **330** or monitoring apparatus via the telephone system. As illustrated in FIG. 3F, the monitoring service is communicatively coupled to the practitioner using any of a number of communication techniques know to those skilled in the art.

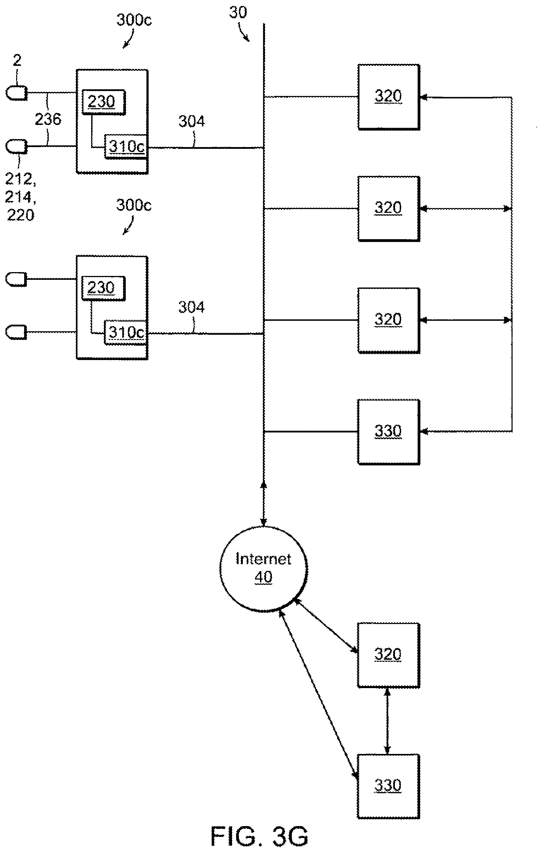

FIG. 3G illustrates a third exemplary device **300***c* that includes an interface **310***c* for communicatively coupling the third exemplary device to a network **30** (e.g., WAN, LAN), which in turn can be communicatively coupled to the internet **40**. In the illustrated embodiment, the interface **310***c* is any of a number of devices as is known to those skilled in the art so that the device can be communicatively coupled via a hard line **304** (e.g., optical, copper, shielded cable).

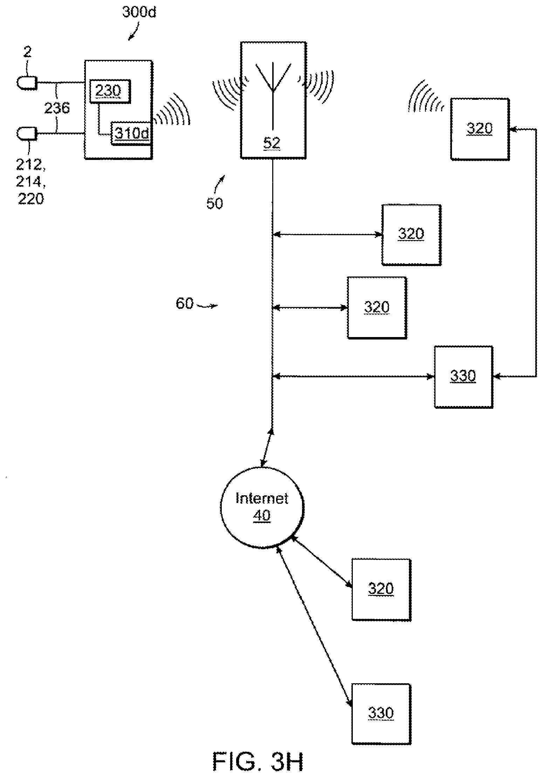

FIG. 3H illustrates a a fourth exemplary device **300***d* including an interface **310***d* that is configured and arranged to communicate with a wireless hub or router **52** so as to thereby create a wireless network **50**. In further embodiments, the wireless network **50** is a part of a larger network **60**. In yet further embodiments, a plurality of devices **300***c* can be coupled to either network **30**, **50**, **60** at any time. Interfaces **310** *c,d* are configured and arranged so that the microprocessor **230** can communicate information over a complimentary network **30**, **50**, **60** to another device that is similarly operably coupled to the network. In this way, the clinical information obtained using either of the devices **300** *c,d* can be communicated to one or both of the practitioners **320** or a monitoring service **330** via the network **30**, **50**, **60**.

As illustrated in FIGS. 3G, H, the monitoring service **330** or monitoring apparatus is communicatively coupled to the practitioner **320** using any of a number of communication techniques know to those skilled in the art. For example, the monitoring apparatus can communicate directly with the practitioner, through the network **30**, **50**, **60** and/or through the internet **40**.

The network can be operably coupled to the internet **40**, so that information from the third or fourth exemplary device **310***c*,*d* is communicated via the network **30**, **50**, **60** and internet **40** to the monitoring service **330** or the practitioner **320**. In the case where the provided device **300***c*,*d* is in a non-clinical setting, for example, the communication interface and device are configurable so that the user can couple the device to the patient's internet connection. The user can be the patient, a person who has no specific medical training or it can be a medically trained person such a visiting nurse or aid. Thus, the acquired information can be transmitted or communicated via that connection to the monitoring service or practitioner.

The foregoing is illustrative of a number of communication techniques; however the foregoing shall not be considered limiting.

As indicated above, the methodology of the present disclosure is illustrated in the flow diagram shown in FIG. 2. In the following discussion, the reference to a system **200** shall be understood to mean a reference to any of the systems described herein. When the clinician intends to assess the cardiac filing pressure of a patient non-invasively using the system **200** of the present invention, the clinician, technician, medical personnel, or patient provides and arranges the optical pulse volume sensing device **210** on a finger **2** or digit of a patient or subject. The optical pulse volume sensing device senses a change in volume caused by the pressure pulse, Step **100**. The medical professional or patient also provides and fluidly couples a pressure transducer **220** to the patient's mouth **4,** so that the pressure transducer thereafter measures expiratory pressure or inspiratory pressure, Step **102**. It should be recognized that these steps can be done in any order.

When so arranged, the optical pulse volume sensing device **210** such as a PPG, senses a change in volume caused by the pressure pulse by illuminating the skin with light from an LED and then measuring the amount of light either transmitted or reflected to a photodiode. The optical pulse volume sensing device **210** provides an output of a pulse pressure signal of cardiac circulatory flow. As also indicated herein, providing and fluidly coupling of the pressure transducer **220** can further include providing a mouth piece and/or tubing, one end of which is fluidly coupled to the patient's mouth. In such a case, the pressure transducer also is disposed in either the mouth piece or tubing so the pressure transducer is remote from the mouth.

After so providing, arranging and coupling the optical pulse volume sensing device **210** and the pressure transducer **220**, pressure data as a function of time or measurements of the patient's pulse amplitude is acquired using the optical pulse volume sensing device **210,** Step **104**. In accordance with the specific directions of the medical procedure controlling the assessment process, the patient is directed to breathe through the mouth piece and/or tubing **212,214** so as to maintain a desired expiratory pressure for a desired period of time, Step **106**. Alternatively, the positive pressure delivery device **280** is controlled so a desired inspiratory pressure is delivered for a predetermined period of time. As indicated herein, a pressure display **260** is provided to assist the patient in maintaining the desired expiratory pressure. While such expiration or positive pressure inspiration is occurring, the expiratory/inspiratory pressure is measured or pressure data is acquired as a function of time using the pressure transducer **220,** Step **108**.

The expiratory or positive pressure inspiratory pressure condition (e.g., desired expiratory pressure) is maintained for a period of 10 or more seconds or 10 or less seconds, or about 10 seconds or in the range of from about 8 to about 12 seconds. The expiratory or positive pressure inspiratory pressure condition is maintained at about 20 mmHg, or at least 20 mmHg, or in the range of from about 20 mmHg to about 35 mmHg, or in the range of from about 10 mmHg to about 50 mmHg. In the case where a plurality of pulse amplitude ratios are to be obtained, (as discussed further herein in connection with Step **110**) and where at least one of the plurality of pulse amplitude ratios is determined under a different expiratory pressure or a different positive pressure inspiratory pressure; each of the different expiratory/positive pressure inspiratory pressures is in the range of from about 10 mmHg to about 50 mmHg. As indicated herein, the pressure display **260** also can include a display of time or elapsed time so that the patient can maintain the desired expiratory pressure for the specified time period.

It is within the scope of the present disclosure to obtain one or more, a plurality or a multiplicity of pressure amplitude ratios under the same expiratory or positive pressure inspiratory pressure conditions or where the expiratory or positive pressure inspiratory pressure conditions differ at least for one of the pulse volume amplitude ratios or per a desired pattern of expiratory pressure conditions. Thus, the specific directions of the medical procedure controlling the assessment process are reviewed initially to determine if more than one pressure amplitude ratio is to be obtained or not. Thus, after the expiratory pressure or positive pressure inspiratory pressure and pulse pressure information or data is acquired, the status of the assessment process is evaluated and a determination is made as to whether the data acquisition process for the intended assessment process is complete, Step **110**. If data acquisition is not complete (No, Step **110**), then the process continues to repeat Steps **104-108**.

If the data acquisition is complete (Yes, Step **110**) then the process proceeds to step **112**, and the calculation or determination of the pulse amplitude ratio is undertaken, where a pulse amplitude ratio is determined for each of the pressure data acquisitions that were made. The pulse amplitude ratio is determined using the pulse amplitude near the end of the expiratory effort and a baseline pulse amplitude for each of the acquired data sets.

The pulse volume of the photoplethysmography waveform, which is the width of the signal from minimum to maximum during one cardiac cycle, from the optical pulse volume sensing device **210** is measured. Also, a comparison is made of the pulse volume near the end of the expiratory effort to the pulse volume at baseline before the expiratory or positive pressure inspiratory effort begins. The ratio of the pulse volume near the end of the expiratory or positive pressure inspiratory effort to the pulse volume before the beginning of the expiratory or positive pressure inspiratory effort, which is called the pulse amplitude ratio, is used.

After determining the pulse amplitude ratio(s), an assessment is made to determine if the corresponding cardiac filling pressure is representative of a normal filling pressure, an elevated filling pressure (generally indicative of a problem) or is in a range which does not provide a reliable indication by itself of an elevated filling pressure condition.

The determined information can be displayed on the output display 250 or via a printing device 240, Step 116. The information being displayed includes but is not limited to the photoplethysmography waveform, the expiratory effort waveform and level, and the pulse amplitude ratio number, as well as words or any combination thereof.

Information that relates to central arterial stiffness can be obtained and assessed. Such methods further include, factoring such arterial stiffness information along with the assessment of the pulse amplitude ratio. A microprocessor or a computer can also be provided. The microprocessor can be communicatively coupled to each of the pressure transducer and the optical pulse volume sensing device (e.g., PPG). The provided microprocessor determines the pulse amplitude ratio from the signals from the pressure transducer and the optical pulse volume sensing device (e.g., PPG).

An applications program including program or code segments and instructions and criteria for carrying out the methods of the present invention, including determining the pulse amplitude ratio on demand, automatically and/or periodically can also be provided. Such an applications program includes program segments and instructions and criteria for causing the periodically determined pulse amplitude ratios as well as any other data such as the pulse amplitude near the end of the expiratory effort, the baseline pulse amplitude and measured expiratory pressures, to be stored in a storage device.

The method further include providing a device including an enclosure, the microprocessor and storage device, where the microprocessor and storage device are disposed within the enclosure so that the device is one of free-standing, on a rolling cart or portable so as for example, the device can be carried in the pocket of a jacket worn by the clinician, technician or medical personnel. The device is arranged so as to include one of a display or a printing device, so that determined information is displayed to the user or printed. This displaying or printing is done one of automatically or in response to an input from the user. The device also can include a means for communicatively coupling the microprocessor to each of the optical pulse pressuring sensing device and the pressure transducer.

A means for communicatively coupling the device to a communication system (e.g., telephone system, network, internet) for communication the acquired information to the practitioner or a service for monitoring for such communications can also be provided. Such monitoring services include storing the acquired information and/or for re-transmitting such information onto the practitioner. Information can be acquired using the provided device in clinical and non-clinical settings and transmitting the acquired information via a communication system to the practitioner and/or monitoring service. In more particular embodiments, such methods further include having a person not having medical training (e.g., patient) use the device to acquiring information, couple the device to a communication system and transmit the so acquired information via the communication system to the practitioner and/or monitoring service.

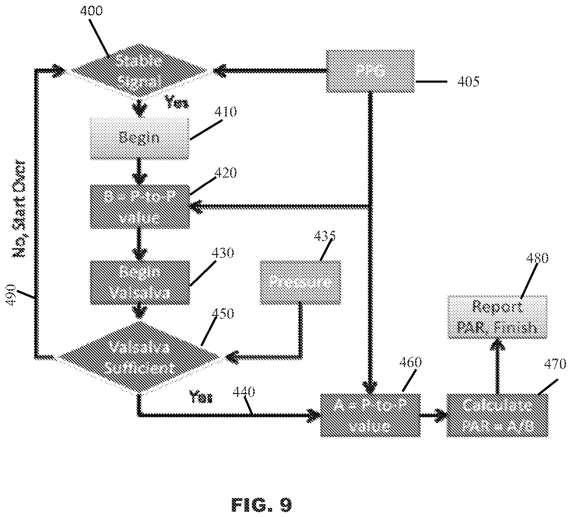

FIG. 9 illustrates a flow diagram representing the automation of the system for non-invasively assessing cardiac filling pressure. Initially the device determines that the PPG signal is stable, step 400. This ensures that the patient isn't moving around and that the results will not be affected by external factors. This is done by determining whether the baseline is within some standard deviation of its mean for a given time (described below). Once the device detects a stable PPG from input 405, it informs the patient that the procedure can begin, step 410. Next, it calculates the baseline peak to peak amplitude, B, step 420. Once the baseline amplitude (B) is detected, it instructs the patient to perform the Valsalva maneuver, step 430 using input 435. As long as the patient holds a proper Valsalva for approximately 10 seconds, condition 440, as determined by the pressure sensor input, the device obtains the second peak to peak value, A, after Valsalva completion, step 460. However, it should be noted that the amount of time for holding the Valsalva maneuver can be any length of time known to one of skill in the art. PAR is then calculated, step 470, and reported as the ratio of A/B, step 480, and LVEDP is predicted from a predefined model.

During Valsalva the software is constantly checking that the patient is performing the maneuver properly. Should the pressure overshoot, fail to reach the goal pressure or otherwise deviate from the boundaries, the device will restart the procedure, condition 490. The particulars of each of these steps are described below.

The central function of the software is to automate the entire procedure including accurate retrieval of the incoming data from the sensors and peak-to-peak detection for the PAR calculation. We implemented a robust serial data detection algorithm in order to handle receiving two sources of input from both the PPG and pressure sensors through the same serial port. Instead of alternating values from the PPG and pressure sensors, two PPG values were followed by two pressure values. The algorithm verifies that the two consecutive values are equal before it considers it a valid datum. This redundancy helps reduce noise and error in the signal.

For peak detection, the function iterates through the array of values that is the PPG signal. When it finds the next value that is less than the previous value, it assumes that the previous value was a local maximum. Similarly, if it finds that the next value is greater than the previous value, this corresponds to a local minimum. There is a heuristically determined 'delta' parameter used to help discriminate between insignificant local extrema.

The software also implements a careful flow control to insure that little involvement on the part of the patient and physician are required. As described above with respect to FIG. 9, the algorithm will continuously read in data from both the PPG and pressure signals after the Start button is pushed. When the Measure button is pushed, it initiates the sequence of automatically detecting the PPG peak-to-peak average to determine if a baseline is reached. If it detects a valid baseline, then it will let the patient know to begin the Valsalva maneuver for 10 seconds. If the Valsalva is done incorrectly or the Restart or Reset buttons are pushed, the entire procedure starts over and detects a new baseline value. After a successful completion of the Valsalva maneuver, the PAR is calculated and displayed. Algorithms were implemented in MATLAB™ and compiled down to stand alone executable for use on the device.

The graphical user interface of the automated device can include two parts: a physician view and a patient view. The physician view displays the PPG signal, the pressure signal, the PAR, and the LVEDP status. Since observing the PPG signal may negatively affect readings, it is hidden on the patient view. The patient sees only two bars: a time progress bar that tells a patient how much longer he must hold the Valsalva maneuver and a pressure status bar that indicates the real-time pressure the patient is exerting. The patient merely has to exert enough pressure to keep the pressure indicator within the desired range, as represented in the visual display for the user. Once the patient completes the procedure, the physician has the option to view the recorded PPG signal. The touch screen allows for an intuitive interface with large buttons and flexible menus that allow the doctor to select various procedure parameters. The fonts are large and easy to read.

In addition, a brief tutorial video explaining how to use the device is included under the help button. The mouthpiece that the patient breathes into is disposable, cheap, and has a one way valve. Its form factor is appropriate for this application and is the same as the kind used in flow meters.

By way of example, patient testing was done using the automated device. The protocol described below for this testing is merely exemplary, and any suitable protocol known to one of skill in the art could be used. The patient testing protocol involved asking hospital patients scheduled for catheterizations (for reasons unrelated to the device) simply to use the device a number of times according to the protocol. In this manner, the PAR values over multiple runs could be compared against the highly accurate catheter LVEDP. Initial results were very favorable. From the data gathered, the device has commendable precision, with small standard deviations for patients A and B over multiple trials. Furthermore, among all of the patients, no PAR values greater than 0.8 were detected. Which is consistent with the fact that from the catheter, no instances of elevated LVEDP (>25 mmHg) were detected. Although more data is necessary, low PAR values seem to correspond to nonelevated LVEDP, as predicted.

There were also several interesting qualitative observations. The patients had very little trouble using the device; by the second or third try they were using the device and performing the Valsalva maneuver properly. One of the patients had chronic obstructive pulmonary disease and he had no trouble holding 20 mmHg for 10 seconds. As designed, individual trials were very fast, 20 to 30 seconds per; in a procedure averaging over multiple trials, the entire process would take under 5 minutes. Lastly, using the doctor's view (where the patient can see his own PPG) didn't affect the outcomes of the results as compared with the patient's view.

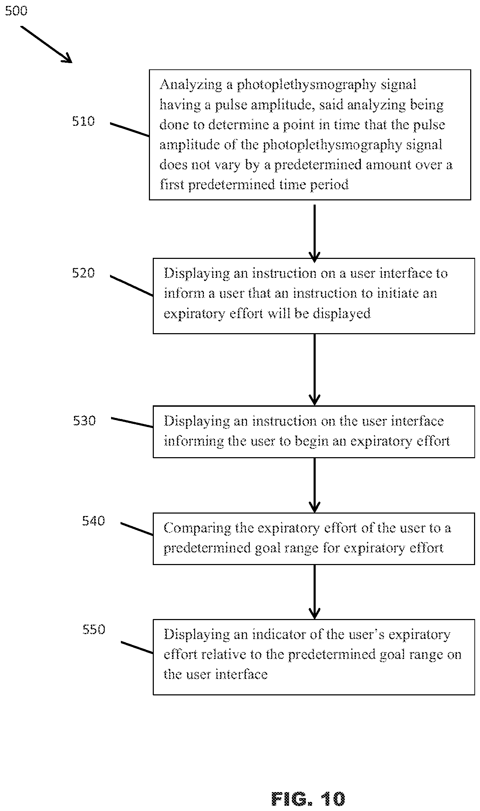

FIG. 10 illustrates a diagram of a method of automating the process of assessing cardiac filling pressure non-invasively. The following method steps can be programmed into a computer readable medium, computer or any other device or system for executing the steps known to one of skill in the art. A method 500 for automating a process for determining cardiac filling pressure non-invasively includes step 510 of analyzing a photoplethysmography signal having a pulse amplitude. The analysis of step 510 is done to determine a point in time that the pulse amplitude of the photoplethysmography signal does not vary by a predetermined amount over a first predetermined time period. Step 520 includes displaying an instruction on a user interface to inform a user that an instruction to initiate an expiratory effort will be displayed, and step 530 includes displaying an instruction on the user interface informing the user to begin an expiratory effort. In step 540, the expiratory effort of the user is compared to a predetermined goal range for expiratory effort. Additionally, in step 550, an indicator of the user's expiratory effort relative to the predetermined goal range is displayed on the user interface.

The method 500 illustrated in FIG. 10 can also include instructing the user to sustain the expiratory effort for a second predetermined period of time, instructing the user to sustain the expiratory effort for a second predetermined period of time. The method can also include displaying an elapsed time for the expiratory effort as well as a time remaining in the second predetermined period of time. It is indicated to the user that the data acquisition sequence is invalid, when the expiratory effort does not meet the predetermined goal range within a third predetermined period of time. It can also be indicated to the user that the data acquisition sequence is invalid when the expiratory effort exceeds the predetermined range for expiratory effort. After the predetermined amount of time has elapsed it is displayed that the user can cease the expiratory effort. A first average of the pulse amplitude of the photoplethysmography signal is calculated for a fourth predetermined period of time before the user ceases the expiratory effort. A second average of the pulse amplitude of the photoplethysmograpy signal is also calculated for a fifth period of time before an initiation of the expiratory effort. Additionally, a pulse amplitude ratio of the first average of the pulse amplitude to the second average of the pulse amplitude is calculated. These steps can also be programmed into the computer, computer readable medium or any other device for executing the steps known to one of skill in the art.

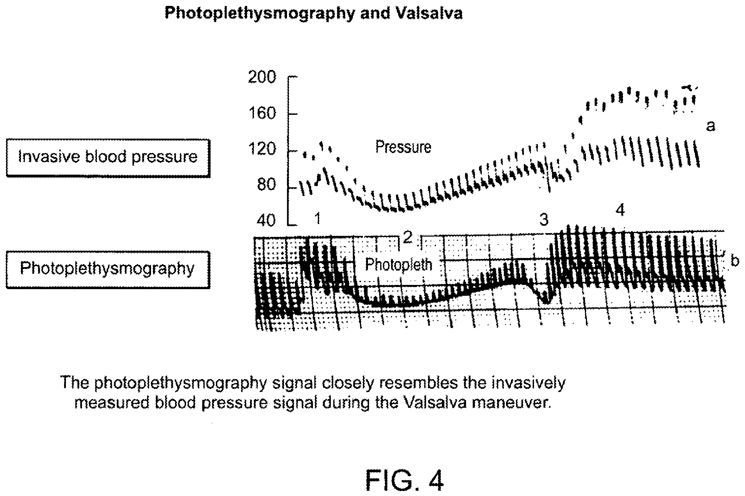

FIG. 4 illustrates a graphical view of a photoplethysmography signal and of invasive blood pressure during the Valsava maneuver.

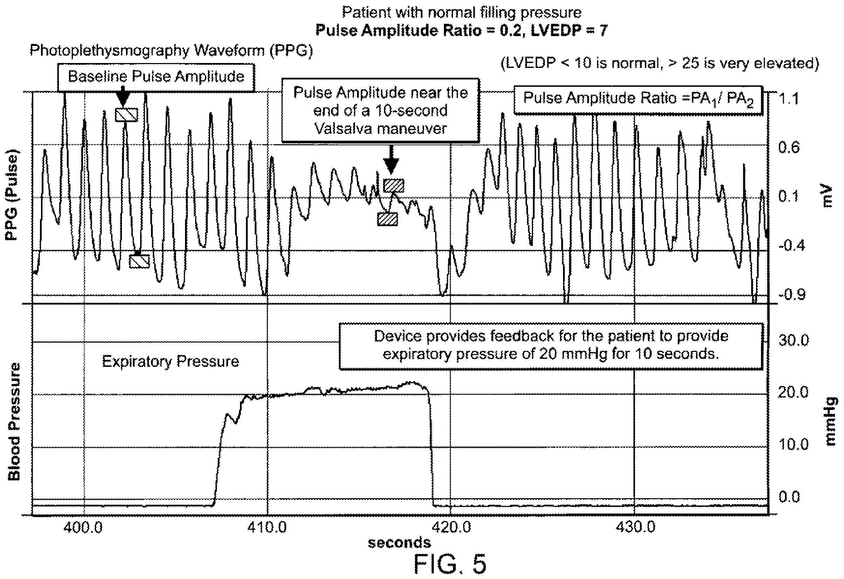

FIG. 5 illustrates a graphical view of a photoplethysmography waveform and the expiratory pressure waveform for a heart with a normal filling pressure. As shown therein, using the methods and systems of the present invention, the determined pulse amplitude ratio equals 0.2 and the LVEDP equals 7.

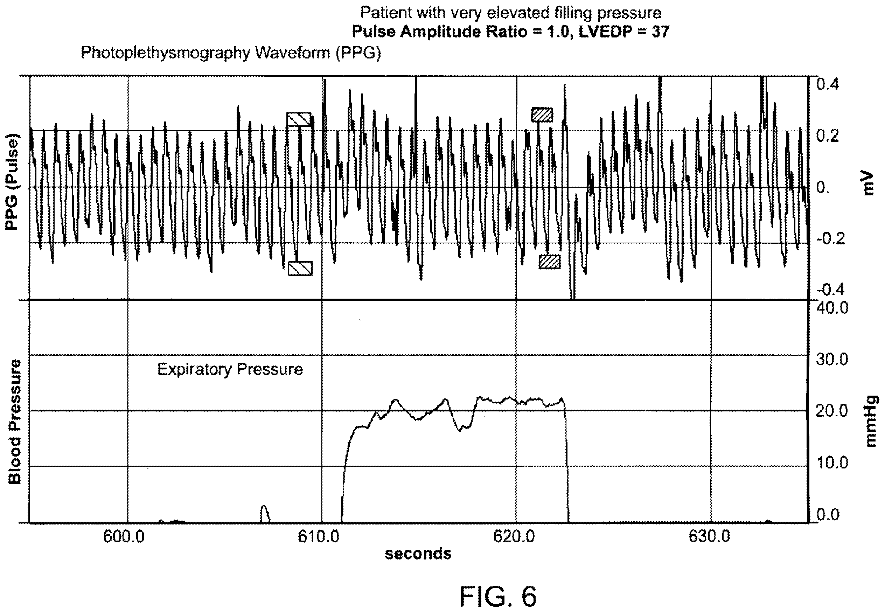

FIG. 6 illustrates a graphical view of a photoplethysmography waveform and the expiratory pressure waveform for a heart with an elevated filling pressure using the system of the present invention. As shown therein, using the methods and systems of the present invention, the determined pulse amplitude ratio equals 1.0 and the LVEDP equals 37.

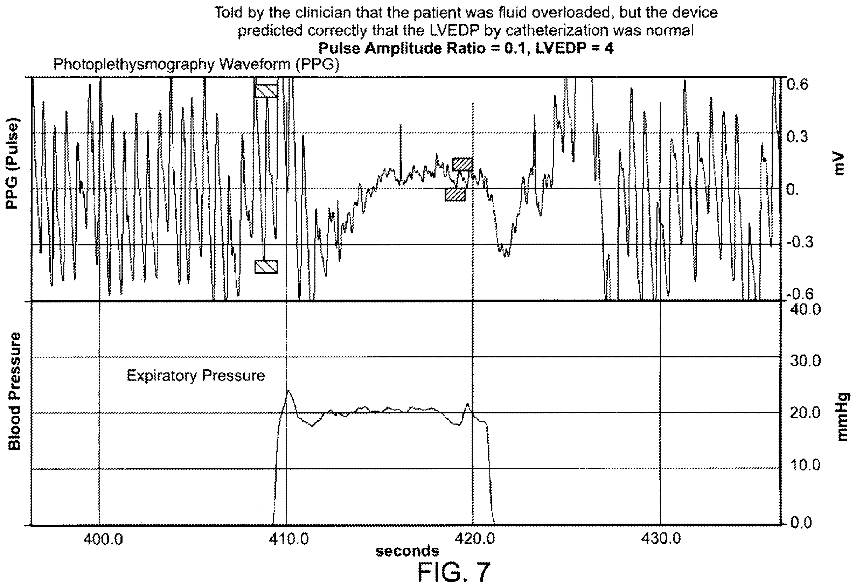

FIG. 7 illustrates a graphical view of a photoplethysmography waveform and the expiratory pressure waveform for the heart of a patient that had been initially diagnosed as being fluid overloaded, but which was later determined that the heart had a normal filling pressure using the system of the present invention. As shown therein, using the methods and systems of the present invention, the determined pulse amplitude ratio equals 0.1 and the LVEDP equals 4.

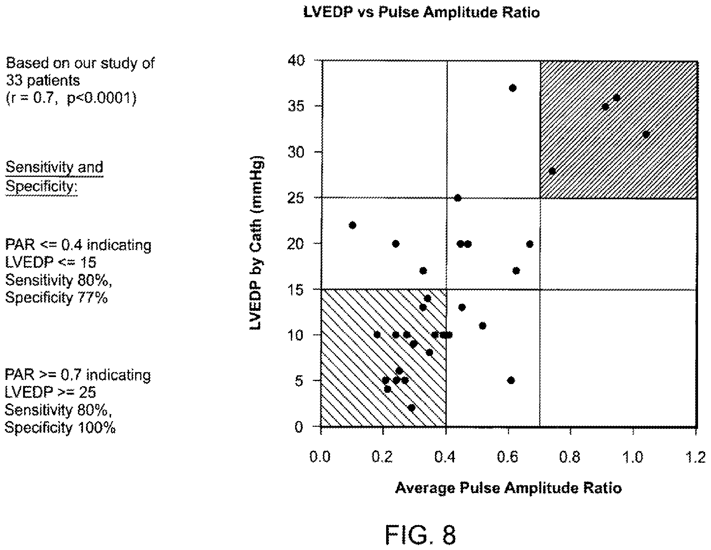

FIG. 8 illustrates a graphical view of LVEDP by catheter versus Average Pulse Amplitude Ratio for a patient study, where the pulse amplitude ratios were determined using the system of the present invention.

Although a preferred embodiment of the invention has been described using specific terms, such description is for illustrative purposes only, and it is to be understood that changes and variations may be made without departing from the scope of the following claims. While this method has been described for use in determining cardiac filling pressure non-invasively, it need not be limited to this application and could be used for any other suitable purpose known to one of skill in the art. The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, the scope of the invention is defined by the appended claims.

## Claims

1. A method of determining a photoplethysmography pulse amplitude ratio for use in automating a process for determining cardiac filling pressure non-invasively and, wherein the method is implemented on a device comprising a user interface and a microprocessor being communicatively coupled to each of an optical pulse volume sensing device and a pressure transducer, the method comprising the following method steps:
analyzing (510) a photoplethysmography signal having a pulse amplitude obtained from the optical pulse volume sensing device (210) said analyzing being done to determine that a variation of a baseline of the photoplethysmography signal is within some standard deviation of its mean over a first predetermined time period before an expiratory effort or positive pressure inspiration;
displaying a first instruction (410, 520) on the user interface to inform a user that a second instruction to initiate an expiratory effort or positive pressure inspiration will be displayed;
displaying the second instruction (430, 530) on the user interface informing the user to begin an expiratory effort or positive pressure inspiration and to sustain the expiratory effort or positive pressure inspiration for a second predetermined period of time;
obtaining the expiratory effort or positive pressure inspiration from the pressure transducer (220);
comparing (450, 540) the expiratory effort or positive pressure inspiration to a predetermined goal range for expiratory effort or positive pressure inspiration and determining (440) via an input of the pressure transducer (220) if the expiratory effort or positive pressure inspiration does not meet the predetermined goal range within a third predetermined period of time; and
displaying an indicator (550) of the user's expiratory effort or positive pressure inspiration relative to the predetermined goal range on the user interface comprising indicating to the user that the data acquisition sequence is invalid when the expiratory effort or positive pressure inspiration does not meet the predetermined goal range within the third predetermined period of time;
calculating a first average (A; 460) of the pulse amplitude of the photoplethysmography signal for a fourth predetermined period of time before the user ceases the expiratory effort or positive pressure inspiration;
calculating a second average (B; 420)of the pulse amplitude of the photoplethysmography signal for a fifth period of time before an initiation of the expiratory effort or positive pressure inspiration; and
calculating a pulse amplitude ratio (A/B; 470) of the first average of the pulse amplitude to the second average of the pulse amplitude.

2. The method of claim 1 further comprising:
programming a computer readable medium with instructions to execute the method steps of claim 1.

3. The method of claim 1 or 2 further comprising displaying an elapsed time for the expiratory effort or positive pressure inspiration as well as a time remaining in the second predetermined period of time.

4. The method of claim 1 or 2 further comprising indicating to the user that the data acquisition sequence is invalid when the expiratory effort or positive pressure inspiration exceeds the predetermined range for expiratory effort or positive pressure inspiration.

5. The method of claim 1 or 2 further comprising displaying that the user can cease the expiratory effort or positive pressure inspiration.

6. An apparatus for determining a photoplethysmography pulse amplitude ratio for use in automating a process for determining cardiac filling pressure non-invasively, comprising:
a device including a housing, said device including a user interface disposed within said housing, an expiratory effort or positive pressure inspiration reception apparatus being coupled to said housing, a photoplethysmograph, a microprocessor being communicatively coupled to each of an optical pulse volume sensing device (210) and a pressure transducer (220) and a computer readable medium, wherein said computer readable medium is programmed with steps and the device is configured to execute the steps of the method programmed on the computer readable medium, the method comprising:
analyzing (510) a signal from the photoplethysmograph, said signal having a pulse amplitude obtained from the optical pulse sensing device (210), said analyzing being done to determine that a variation of a baseline of the photoplethysmography signal is within some standard deviation of its mean over a first predetermined time period before an expiratory effort or positive pressure inspiration;
transmitting a first instruction to be displayed (410, 530) on a user interface, said first instruction informing a user that a second instruction to initiate an expiratory effort or positive pressure inspiration will be displayed;
transmitting the second instruction (430, 530) to be displayed on the user interface, said second instruction informing the user to begin an expiratory effort or positive pressure inspiration and to sustain the expiratory effort or positive pressure inspiration for a second predetermined period of time;
obtaining the expiratory effort or positive pressure inspiration from the pressure transducer (220);
comparing (450, 540) the expiratory effort or positive pressure inspiration to a predetermined goal range for expiratory effort or positive pressure inspiration and determining (440) via an input of the pressure transducer (220) if the expiratory effort or positive pressure inspiration does not meet the predetermined goal range within a third predetermined period of time;
transmitting a third instruction to be displayed (550) on the user interface, said third instruction indicating the user's expiratory effort or positive pressure inspiration relative to the predetermined goal range on the user interface comprising indicating to the user that the data acquisition sequence is invalid when the expiratory effort or positive pressure inspiration does not meet the predetermined goal range within the third predetermined period of time;
calculating a first average (A; 460) of the pulse amplitude of the photoplethysmography signal for a fourth predetermined period of time before the user ceases the expiratory effort or positive pressure inspiration;
calculating a second average (B; 420) of the pulse amplitude of the photoplethysmography signal for a fifth period of time before an initiation of the expiratory effort or positive pressure inspiration; and
calculating a pulse amplitude ratio (A/B; 470); of the first average of the pulse amplitude to the second average of the pulse amplitude.

7. The apparatus of claim 6 further comprising said computer readable medium being disposed within the housing of the device.

## Patentansprüche

1. Ein Verfahren zum Bestimmen eines
Photoplethysmographiepulsamplitudenverhältnisses zur Verwendung beim Automatisieren eines Vorgangs zum nicht invasiven Bestimmen von kardialem Füllungsdruck und wobei das Verfahren auf einer Vorrichtung implementiert wird, die eine Benutzerschnittstelle und einen Mikroprozessor, der jeweils mit einer optischen Pulsvolumenmessvorrichtung und einem Druckwandler kommunikativ gekoppelt ist, beinhaltet, wobei das Verfahren die folgenden Verfahrensschritte beinhaltet:
Analysieren (510) eines Photoplethysmographiesignals, das eine Pulsamplitude aufweist, die von der optischen Pulsvolumenmessvorrichtung (210) erhalten wurde, wobei das Analysieren durchgeführt wird, um zu bestimmen, dass eine Abwandlung einer Grundlinie des Photoplethysmographiesignals über einen ersten vorbestimmten Zeitraum vor einer Ausatmungsanstrengung oder Überdruckeinatmung innerhalb einer Standardabweichung seines Mittelwerts liegt;
Darstellen einer ersten Anweisung (410, 520) auf der Benutzerschnittstelle, um einen Benutzer zu informieren, dass eine zweite Anweisung zum Einleiten einer Ausatmungsanstrengung oder Überdruckeinatmung dargestellt werden wird;
Darstellen der zweiten Anweisung (430, 530) auf der Benutzerschnittstelle, was den Benutzer informiert, eine Ausatmungsanstrengung oder Überdruckeinatmung zu beginnen und die Ausatmungsanstrengung oder Überdruckeinatmung für einen zweiten vorbestimmten Zeitraum aufrechtzuerhalten;
Erhalten der Ausatmungsanstrengung oder Überdruckeinatmung von dem Druckwandler (220);
Vergleichen (450, 540) der Ausatmungsanstrengung oder Überdruckeinatmung mit einem vorbestimmten Zielbereich für Ausatmungsanstrengung oder
Überdruckeinatmung und Bestimmen (440), über eine Eingabe des Druckwandlers (220), ob die Ausatmungsanstrengung oder Überdruckeinatmung innerhalb eines dritten vorbestimmten Zeitraums nicht den vorbestimmten Zielbereich erreicht; und
Darstellen einer Anzeige (550) der Ausatmungsanstrengung oder Überdruckeinatmung des Benutzers relativ zu dem vorbestimmten Zielbereich auf der Benutzerschnittstelle, was das Anzeigen für den Benutzer beinhaltet, dass die Datengewinnungssequenz ungültig ist, wenn die Ausatmungsanstrengung oder Überdruckeinatmung innerhalb des dritten vorbestimmten Zeitraums nicht den vorbestimmten Zielbereich erreicht;
Berechnen eines ersten Durchschnitts (A; 460) der Pulsamplitude des Photoplethysmographiesignals für einen vierten vorbestimmten Zeitraum, bevor der Benutzer die Ausatmungsanstrengung oder Überdruckeinatmung beendet;
Berechnen eines zweiten Durchschnitts (B; 420) der Pulsamplitude des Photoplethysmographiesignals für einen fünften Zeitraum vor einer Einleitung der Ausatmungsanstrengung oder Überdruckeinatmung; und
Berechnen eines Pulsamplitudenverhältnisses (A/B; 470) des ersten Durchschnitts der Pulsamplitude zu dem zweiten Durchschnitt der Pulsamplitude.

2. Verfahren gemäß Anspruch 1, das ferner Folgendes beinhaltet:
Programmieren eines computerlesbaren Mediums mit Anweisungen, um die Verfahrensschritte gemäß Anspruch 1 auszuführen.

3. Verfahren gemäß Anspruch 1 oder 2, das ferner das Darstellen einer abgelaufenen Zeit für die Ausatmungsanstrengung oder Überdruckeinatmung sowie einer verbleibenden Zeit in dem zweiten vorbestimmten Zeitraum beinhaltet.

4. Verfahren gemäß Anspruch 1 oder 2, das ferner das Anzeigen für den Benutzer beinhaltet, dass die Datengewinnungssequenz ungültig ist, wenn die Ausatmungsanstrengung oder Überdruckeinatmung den vorbestimmten Bereich für Ausatmungsanstrengung oder Überdruckeinatmung überschreitet.

5. Verfahren gemäß Anspruch 1 oder 2, das ferner das Darstellen beinhaltet, dass der Benutzer die Ausatmungsanstrengung oder Überdruckeinatmung beenden kann.

6. Ein Apparat zum Bestimmen eines Photoplethysmographiepulsamplitudenverhältnisses zur Verwendung beim Automatisieren eines Vorgangs zum nicht invasiven Bestimmen eines kardialen Füllungsdrucks, der Folgendes beinhaltet:
eine Vorrichtung, die ein Gehäuse umfasst, wobei die Vorrichtung eine Benutzerschnittstelle, die innerhalb des Gehäuses angeordnet ist, einen Ausatmungsanstrengungs- oder Überdruckeinatmungsempfangsapparat, der mit dem Gehäuse gekoppelt ist, einen Photoplethysmograph, einen Mikroprozessor, der jeweils mit einer optischen Pulsvolumenmessvorrichtung (210) und einem Druckwandler (220) gekoppelt ist, und ein computerlesbares Medium umfasst, wobei das computerlesbare Medium mit Schritten programmiert ist und die Vorrichtung konfiguriert ist, um die Schritte des Verfahrens auszuführen, die auf dem computerlesbaren Medium programmiert sind, wobei das Verfahren Folgendes beinhaltet:
Analysieren (510) eines Signals von dem Photoplethysmograph, wobei das Signal eine Pulsamplitude aufweist, die von der optischen Pulsvolumenmessvorrichtung (210) erhalten wurde, wobei das Analysieren durchgeführt wird, um zu bestimmen, dass eine Abwandlung einer Grundlinie des Photoplethysmographiesignals über einen ersten vorbestimmten Zeitraum vor einer Ausatmungsanstrengung oder Überdruckeinatmung innerhalb einer Standardabweichung seines Mittelwerts liegt;
Übertragen einer ersten auf einer Benutzerschnittstelle darzustellenden (410, 530) Anweisung, wobei die erste Anweisung einen Benutzer informiert, dass eine zweite Anweisung zum Einleiten einer Ausatmungsanstrengung oder Überdruckeinatmung dargestellt werden wird;
Übertragen der zweiten auf der Benutzerschnittstelle darzustellenden Anweisung (430, 530), wobei die zweite Anweisung den Benutzer informiert, eine Ausatmungsanstrengung oder Überdruckeinatmung zu beginnen und die Ausatmungsanstrengung oder Überdruckeinatmung für einen zweiten vorbestimmten Zeitraum aufrechtzuerhalten;
Erhalten der Ausatmungsanstrengung oder Überdruckeinatmung von dem Druckwandler (220);
Vergleichen (450, 540) der Ausatmungsanstrengung oder Überdruckeinatmung mit einem vorbestimmten Zielbereich für Ausatmungsanstrengung oder
Überdruckeinatmung und Bestimmen (440), über eine Eingabe des Druckwandlers (220), ob die Ausatmungsanstrengung oder Überdruckeinatmung innerhalb eines dritten vorbestimmten Zeitraums nicht den vorbestimmten Zielbereich erreicht;
Übertragen einer dritten auf der Benutzerschnittstelle darzustellenden (550) Anweisung,
wobei die dritte Anweisung die Ausatmungsanstrengung oder Überdruckeinatmung des Benutzers relativ zu dem vorbestimmten Zielbereich auf der Benutzerschnittstelle anzeigt, was das Anzeigen für den Benutzer beinhaltet, dass die Datengewinnungssequenz ungültig ist, wenn die Ausatmungsanstrengung oder
Überdruckeinatmung innerhalb des dritten vorbestimmten Zeitraums nicht den vorbestimmten Zielbereich erreicht;
Berechnen eines ersten Durchschnitts (A; 460) der Pulsamplitude des Photoplethysmographiesignals für einen vierten vorbestimmten Zeitraum, bevor der Benutzer die Ausatmungsanstrengung oder Überdruckeinatmung beendet;
Berechnen eines zweiten Durchschnitts (B; 420) der Pulsamplitude des Photoplethysmographiesignals für einen fünften Zeitraum vor einer Einleitung der Ausatmungsanstrengung oder Überdruckeinatmung; und
Berechnen eines Pulsamplitudenverhältnisses (A/B; 470) des ersten Durchschnitts der Pulsamplitude zu dem zweiten Durchschnitt der Pulsamplitude.

7. Apparat gemäß Anspruch 6, der ferner beinhaltet, dass das computerlesbare Medium innerhalb des Gehäuses der Vorrichtung angeordnet ist.

## Revendications

1. Une méthode de détermination d'un rapport d'amplitude d'impulsion de photopléthysmographie destiné à une utilisation dans l'automatisation d'un procédé pour déterminer une pression de remplissage cardiaque de manière non invasive, et où la méthode est mise en œuvre sur un dispositif comprenant une interface utilisateur et un microprocesseur étant couplés de manière à communiquer à chaque élément parmi un dispositif de détection de volume d'impulsion optique et un transducteur de pression, la méthode comprenant les étapes de méthode suivantes :
l'analyse (510) d'un signal de photopléthysmographie ayant une amplitude d'impulsion obtenue à partir du dispositif de détection de volume d'impulsion optique (210), ladite analyse étant réalisée pour déterminer qu'une variation d'une ligne de base du signal de photopléthysmographie est dans les limites d'un certain écart-type de sa moyenne sur un premier laps de temps prédéterminé avant un effort expiratoire ou une inspiration en pression positive ;
l'affichage d'une première instruction (410, 520) sur l'interface utilisateur pour informer un utilisateur qu'une deuxième instruction de commencer un effort expiratoire ou une inspiration en pression positive va être affichée ;
l'affichage de la deuxième instruction (430, 530) sur l'interface utilisateur informant l'utilisateur d'amorcer un effort expiratoire ou une inspiration en pression positive et de maintenir l'effort expiratoire ou l'inspiration en pression positive pendant un deuxième laps de temps prédéterminé ;
l'obtention de l'effort expiratoire ou de l'inspiration en pression positive à partir du transducteur de pression (220) ;
la comparaison (450, 540) de l'effort expiratoire ou de l'inspiration en pression positive à un intervalle cible prédéterminé pour l'effort expiratoire ou l'inspiration en pression positive et la détermination (440) par l'intermédiaire d'une entrée du transducteur de pression (220) quant à savoir si l'effort expiratoire ou l'inspiration en pression positive ne satisfait pas à l'intervalle cible prédéterminé dans les limites d'un troisième laps de temps prédéterminé ; et
l'affichage d'un indicateur (550) de l'effort expiratoire ou de l'inspiration en pression positive de l'utilisateur relativement à l'intervalle cible prédéterminé sur l'interface utilisateur comprenant l'indication à l'utilisateur du fait que la séquence d'acquisition de données est invalide lorsque l'effort expiratoire ou l'inspiration en pression positive ne satisfait pas à l'intervalle cible prédéterminé dans les limites du troisième laps de temps prédéterminé ;
le calcul d'une première moyenne (A ; 460) de l'amplitude d'impulsion du signal de photopléthysmographie pendant un quatrième laps de temps prédéterminé avant que l'utilisateur ne cesse l'effort expiratoire ou l'inspiration en pression positive ;
le calcul d'une deuxième moyenne (B ; 420) de l'amplitude d'impulsion du signal de photopléthysmographie pendant un cinquième laps de temps avant un commencement de l'effort expiratoire ou de l'inspiration en pression positive ; et
le calcul d'un rapport d'amplitude d'impulsion (A/B ; 470) de la première moyenne de l'amplitude d'impulsion à la deuxième moyenne de l'amplitude d'impulsion.

2. La méthode de la revendication 1 comprenant en outre :
la programmation d'un support lisible par ordinateur avec des instructions visant à exécuter les étapes de méthode de la revendication 1.

3. La méthode de la revendication 1 ou de la revendication 2 comprenant en outre l'affichage d'un temps écoulé pour l'effort expiratoire ou l'inspiration en pression positive de même que d'un temps restant dans le deuxième laps de temps prédéterminé.

4. La méthode de la revendication 1 ou de la revendication 2 comprenant en outre l'indication à l'utilisateur du fait que la séquence d'acquisition de données est invalide lorsque l'effort expiratoire ou l'inspiration en pression positive dépasse l'intervalle prédéterminé pour l'effort expiratoire ou l'inspiration en pression positive.

5. La méthode de la revendication 1 ou de la revendication 2 comprenant en outre l'affichage du fait que l'utilisateur peut cesser l'effort expiratoire ou l'inspiration en pression positive.

6. Un appareil pour déterminer un rapport d'amplitude d'impulsion de photopléthysmographie destiné à une utilisation dans l'automatisation d'un procédé pour déterminer une pression de remplissage cardiaque de manière non invasive, comprenant :
un dispositif incluant un logement, ledit dispositif incluant une interface utilisateur disposée à l'intérieur dudit logement, un appareil de réception d'effort expiratoire ou
d'inspiration en pression positive étant couplé audit logement, un photopléthysmographe, un microprocesseur étant couplé de manière à communiquer à chaque élément parmi un dispositif de détection de volume d'impulsion optique (210) et
un transducteur de pression (220) et un moyen lisible par ordinateur, où ledit moyen lisible par ordinateur est programmé avec des étapes et le dispositif est configuré pour exécuter les étapes de la méthode programmées sur le moyen lisible par ordinateur, la méthode comprenant :
l'analyse (510) d'un signal en provenance du photopléthysmographe, ledit signal ayant une amplitude d'impulsion obtenue à partir du dispositif de détection d'impulsion optique (210), ladite analyse étant réalisée pour déterminer qu'une variation d'une ligne de base du signal de photopléthysmographie est dans les limites d'un certain écart-type de sa moyenne sur un premier laps de temps prédéterminé avant un effort expiratoire ou une inspiration en pression positive ;
la transmission d'une première instruction devant être affichée (410, 530) sur une interface utilisateur, ladite première instruction informant un utilisateur qu'une deuxième instruction de commencer un effort expiratoire ou une inspiration en pression positive va être affichée ;
la transmission de la deuxième instruction (430, 530) devant être affichée sur l'interface utilisateur, ladite deuxième instruction informant l'utilisateur d'amorcer un effort expiratoire ou une inspiration en pression positive et de maintenir l'effort expiratoire ou
l'inspiration en pression positive pendant un deuxième laps de temps prédéterminé ;
l'obtention de l'effort expiratoire ou de l'inspiration en pression positive à partir du transducteur de pression (220) ;
la comparaison (450, 540) de l'effort expiratoire ou de l'inspiration en pression positive à un intervalle cible prédéterminé pour l'effort expiratoire ou l'inspiration en pression positive et la détermination (440) par l'intermédiaire d'une entrée du transducteur de pression (220) quant à savoir si l'effort expiratoire ou l'inspiration en pression positive ne satisfait pas à l'intervalle cible prédéterminé dans les limites d'un troisième laps de temps prédéterminé ;
la transmission d'une troisième instruction devant être affichée (550) sur l'interface utilisateur, ladite troisième instruction indiquant l'effort expiratoire ou l'inspiration en pression positive de l'utilisateur relativement à l'intervalle cible déterminé sur l'interface utilisateur comprenant l'indication à l'utilisateur du fait que la séquence d'acquisition de données est invalide lorsque l'effort expiratoire ou l'inspiration en pression positive ne satisfait pas à l'intervalle cible prédéterminé dans les limites du troisième laps de temps prédéterminé ;
le calcul d'une première moyenne (A ; 460) de l'amplitude d'impulsion du signal de photopléthysmographie pendant un quatrième laps de temps prédéterminé avant que l'utilisateur ne cesse l'effort expiratoire ou l'inspiration en pression positive ;
le calcul d'une deuxième moyenne (B ; 420) de l'amplitude d'impulsion du signal de photopléthysmographie pendant un cinquième laps de temps avant un commencement de l'effort expiratoire ou de l'inspiration en pression positive ; et
le calcul d'un rapport d'amplitude d'impulsion (A/B ; 470) de la première moyenne de l'amplitude d'impulsion à la deuxième moyenne de l'amplitude d'impulsion.

7. L'appareil de la revendication 6 comprenant en outre ledit support lisible par ordinateur étant disposé à l'intérieur du logement du dispositif.
